# EUROPÄISCHE PATENTANMELDUNG

(11) **EP 4 183 332 A1**
(43) Veröffentlichungstag der Anmeldung: **24.05.2023**
(21) Anmeldenummer: 21209846.1
(22) Anmeldetag: 23.11.2021
(51) Int. Cl.: A61B 5/055, A61B 6/00, G06T 7/136, A61B 6/03

(54) **VERFAHREN ZUR VERBESSERTEN THERAPIEPLANUNG MITTELS EINES MAGNETRESONANZTOMOGRAPHEN**

(71) Anmelder: Siemens Healthcare GmbH, 91052 Erlangen (DE)
(72) Erfinder: Möhler, Christian, 76131 Karlsruhe (DE); Requardt, Martin, 90425 Nürnberg (DE)

(57) **Zusammenfassung**

Die Erfindung betrifft ein Verfahren zur Therapieplanung für eine Strahlentherapie mittels eines Magnetresonanztomographen und eines Computertomographen. Es werden eine Computertomographie und eine Magnetresonanztomographie eines Patienten bereitgestellt, Knochen des Patienten in den Abbildungen segmentiert und in der Magnetresonanztomographie registriert. Eine synthetische Computertomographie wird anhand der Magnetresonanztomographie in Abhängigkeit der registrierten Knochen erzeugt und damit ein Therapieplan erstellt.

## Beschreibung

Die Erfindung betrifft ein Verfahren zur verbesserten Therapieplanung für eine Strahlentherapie mit einem Magnetresonanztomographen.

Magnetresonanztomographen sind bildgebende Vorrichtungen, die zur Abbildung eines Untersuchungsobjektes Kernspins des Untersuchungsobjektes mit einem starken äußeren Magnetfeld ausrichten und durch ein magnetisches Wechselfeld zur Präzession um diese Ausrichtung anregen. Die Präzession bzw. Rückkehr der Spins aus diesem angeregten in einen Zustand mit geringerer Energie wiederum erzeugt als Antwort ein magnetisches Wechselfeld, das über Antennen empfangen wird.

Mit Hilfe von magnetischen Gradientenfeldern wird den Signalen eine Ortskodierung aufgeprägt, die nachfolgend eine Zuordnung von dem empfangenen Signal zu einem Volumenelement ermöglicht. Das empfangene Signal wird dann ausgewertet und eine dreidimensionale bildgebende Darstellung des Untersuchungsobjektes bereitgestellt. Zum Empfang des Signals werden vorzugsweise lokale Empfangsantennen, sogenannte Lokalspulen verwendet, die zur Erzielung eines besseren Signal-Rauschabstandes unmittelbar am Untersuchungsobjekt angeordnet werden. Die Empfangsantennen können auch in einer Patientenliege verbaut sein.

In der Therapieplanung für eine Strahlentherapie wird üblicherweise ein Computertomographieaufnahme genutzt, die eine dreidimensionale Wiedergabe eines zu therapierenden Bereichs eines Patienten liefert. Die Computertomographie, hier im speziellen die sogenannte Cone-Beam-Röntgentomographie (CBCT), liefert dabei ein geometrisch korrektes Abbild, das aufgrund der geradlinigen Ausbreitung der Röntgenstrahlen so gut wie keine geometrischen Verzerrungen aufweist.

Gleichzeitig sind die Fähigkeiten der Computertomographie mit Röntgenstrahlung jedoch bei der Abbildung von Weichteilen sehr begrenzt, da diese nur jeweils geringe Dichteunterschiede aufweisen. Ein Tumor lässt sich so nur schwer von dem umgebenden Gewebe abgrenzen.

Dies ist wiederum eine Stärke der Magnetresonanztomographie, die eine besonders kontrastreiche Darstellung unterschiedlicher Gewebe und Organe liefert. Darüber hinaus verzichtet die Magnetresonanztomographie auf ionisierende Strahlung und kann ohne zusätzliches Risiko für den Patienten eingesetzt werden. Es wird daher zunehmend eine Therapieplanung mittels Magnetresonanzaufnahmen vorgenommen. Dabei wird aus den Magnetresonanztomographen eine Schwächung und oder Absorption eines zur Therapie verwendeten Strahls aus hochenergetischen Photonen oder Partikeln unter Annahme von Absorptionskoeffizienten des jeweiligen Gewebes ermittelt.

Wegen des ähnlichen Verhaltens von den zur Therapie verwendeten Strahlen und den Röntgenstrahlen bei der Computertomographie wurden dafür in der Vergangenheit die Computertomographien verwendet. In Analogie dazu werden zur Abschätzung der Abschwächung aus den Magnetresonanzdaten sogenannte synthetische CT (Computertomographie) errechnet, um auch die bisher gewohnten Arbeitsabläufe und Prozesse nutzen zu können. Die Annahmen über die Abschwächung durch das Gewebe kann aber nicht individuell unterschiedliche Eigenschaften wiedergeben. Es ist daher eine Aufgabe der Erfindung, die Therapieplanung mit Magnetresonanztomographen zu verbessern.

Diese Aufgabe wird von einem Verfahren nach Anspruch 1 gelöst.

Das erfindungsgemäße Verfahren ist zur Therapieplanung für eine Strahlentherapie unter Verwendung eines Magnetresonanztomographen, d.h. mittels des Magnetresonanztomographen erfassten Abbildungen vorgesehen. Das Verfahren wird vorzugsweise ganz oder teilweise auf einer programmierbaren Steuerung ausgeführt. Die Steuerung kann beispielsweise auch die Steuerung eines Magnetresonanztomographen sein. Denkbar ist auch ein über ein Datennetzwerk verbundene Mehrzahl an Steuerungen, beispielsweise auch als cloudbasierter Dienst.

In einem Schritt des erfindungsgemäßen Verfahrens wird eine Computertomographie eines zu therapierenden Bereichs eines Patienten der Steuerung bereitgestellt. Als Computertomographie wird eine Abbildung des Patienten mittels eines Computertomographen bezeichnet. Das Bereitstellen kann das Einlesen einer bereits in der Vergangenheit aufgenommenen Computertomographie des Patienten sein, die auf einem Datenträger bereitgestellt wird oder von einem Netzwerkspeicher heruntergeladen wird. Es ist aber auch denkbar, dass eine Computertomographie beispielsweise mit einem CBCT neu erfasst wird. Als Computertomographie wird hier insbesondere eine Abbildung verstanden, die mittels Röntgenstrahlen eine dreidimensionale Information über eine Abschwächung von Röntgenstrahlen liefert.

Als zu therapierender Bereich wird dabei im Sinne der Erfindung nicht nur der Bereich angesehen, in dem die Strahlentherapie durch die ionisierende Strahlung ihre Wirkung erzielen soll, also insbesondere der Tumor, sondern auch alle Bereiche bzw. Gewebe, die die ionisierende Strahlung auf dem Weg zu ihrem Ziel, dem Tumor, durchstrahlt.

In einem weiteren Schritt des erfindungsgemäßen Verfahrens wird eine Magnetresonanztomographie, d.h. eine dreidimensionale Abbildung des zu therapierenden Bereichs mittels des Magnetresonanztomographen erfasst. Dabei bilden die Computertomographie und die Magnetresonanztomographie beide zumindest das Ziel, z.B. den Tumor, umgebenden Gewebe und insbesondere Knochen in beiden Modalitäten ab.

In einem anderen Schritt des erfindungsgemäßen Verfahrens werden Knochen in der Computertomographie segmentiert. Unter Knochen im Sinne der der Erfindung werden alle Gewebe bzw. auch Gegenstände wie Implantate angesehen, die ein einem Knochen vergleichbare oder höhere Abschwächungskoeffizienten für Röntgenstrahlen oder ionisierende Strahlen aufweisen. Es kann hierbei ein vorbestimmter Grenzwert für den Abschwächungskoeffizient vorgegeben sein, oberhalb dessen ein Gewebe oder Material als Knochen im Sinne der Erfindung angesehen wird. Ein beispielhafter Wert wäre 150 HU.

Unter Segmentieren wird hier das Bestimmen der Grenzfläche im dreidimensionalen Raum und/oder Kontur im zweidimensionalen Raum der Knochen zu dem umgebenden Gewebe mit niedrigerem Abschwächungskoeffizienten angesehen. Mit anderen Worten, die Form des Knochens wird in zwei und/oder drei Dimensionen aus der Computertomographie ermittelt. Das Segmentieren erfolgt beispielsweise in der Steuerung, einem dedizierten System oder auch in einer cloudbasierten Anwendung.

In einem weiteren Schritt des erfindungsgemäßen Verfahrens werden die Knochen in der Magnetresonanztomographie segmentiert. Hierbei gilt im Wesentlichen das zuvor gesagte zum Segmentieren der Computertomographie, allerdings kann hier nicht das Kriterium der Abschwächung für ionisierende Strahlung zur Unterscheidung von Knochen und anderem Gewebe angewendet werden, sondern es sind Kriterien erforderlich, die durch die Magnetresonanztomographie erfasst werden können. Denkbar sind hier beispielsweise Wasser- und/oder Fettgehalt des abgebildeten Gewebes oder unterschiedliche Relaxationszeiten der Kernspins.

In einem anderen Schritt des erfindungsgemäßen Verfahrens werden die in der Computertomographie segmentierten Knochen in der Magnetresonanztomographie registriert. Darunter ist zu verstehen, dass eine Zuordnung der in der Computertomographie segmentierten Knochen zu den gleichen segmentierten Knochen in der Magnetresonanztomographie erfolgt. Diese Zuordnung umfasst die Zuordnung mindestens einer Eigenschaft, die in der Computertomografie für den jeweiligen Knochen erfasst wurde, zu dem korrespondierenden Knochen in der Magnetresonanztomographie. Vorzugsweise handelt es sich dabei um eine Eigenschaft, die einen Einfluss auf die Ausbreitung einer ionisierenden Strahlung bei der Therapie haben. Denkbar sind aber auch geometrische Eigenschaften wie Form oder Abmessungen. Die Zuordnung umfasst vorzugsweise auch die Ausrichtung und Position der Knochen. Auch das Registrieren erfolgt beispielsweise in der Steuerung, einem dedizierten System oder auch in einer cloudbasierten Anwendung.

In einem weiteren Schritt wird eine synthetische Computertomographie aus der Magnetresonanztomographie in Abhängigkeit der registrierten Knochen der Computertomographie ermittelt. Unter einer synthetischen Computertomographie wird dabei eine simulierte Computertomographie verstanden, bei der die Abschwächung der Röntgenstrahlen anhand der Magnetresonanztomographie, insbesondere der geometrischen Verteilung der Gewebe, errechnet wird. Beispielsweise kann anhand der Abschwächung des simulierten Strahls unter unterschiedlichen Ausrichtungen bezüglich der virtuellen Fassung des zu untersuchenden Bereichs in der Magnetresonanztomographie mit den Verfahren der Computertomographie eine synthetische Abbildung errechnet werden. Dabei müssen Annahmen über die Abschwächungskoeffizienten der unterschiedlichen Gewebetypen für den Röntgenstrahl gemacht werden. Vorzugsweise wird dabei insbesondere für die Knochen auf Eigenschaften zurückgegriffen, die aus der ursprünglichen realen Computertomographie ermittelt wurden und bei der Registrierung den Knochen in der Magnetresonanztomographie zugeordnet wurden. Es sind aber auch andere Verfahren zur Erzeugung eines synthetischen denkbar und bekannt, beispielsweise auch mit Hilfe einer trainierten künstlichen Intelligenz.

In einem anderen Schritt wird ein Therapieplan in Abhängigkeit von der synthetischen Computertomographie ermittelt. Dieser Schritt ist in der konventionellen Therapieplanung für real am Patienten mittels Röntgenstrahlen erfasste Computertomographien bekannt. Vorzugsweise wird der Therapieplan an einen Nutzer ausgegeben.

Auch das Ermitteln der synthetischen Computertomographie und des Therapieplans können beispielsweise in der Steuerung, einem dedizierten System oder auch in einer cloudbasierten Anwendung erfolgen.

Auf vorteilhafte Weise verbindet das erfindungsgemäße Verfahren die Vorteile beider bildgebender Modalitäten, der Computertomographie und der Magnetresonanztomographie. Mit einer realen Computertomographie, die in dem Verfahren bereitgestellt werden oder erfasst wird, können auf vorteilhafte Weise Daten zur Knochendichte bzw. zur Abschwächung der ionisierenden Strahlung durch die Knochen bereitgestellt werden, die den individuellen Eigenschaften des Patienten entsprechen. Diese Daten ändern sich nicht kurzfristig und können im weiteren Verlauf der Behandlung weiter genutzt werden. Die Magnetresonanztomographie erfasst insbesondere Tumorgewebe besser und führt durch den Verzicht auf ionisierende Strahlung zu keiner weiteren Belastung des Patienten. So kann zu jedem Zeitpunkt eine aktuelle Lage der Gewebe und Knochen erfasst werden und dennoch ein möglichst genauer Behandlungsplan erstellt werden. Aufgrund der Verwendung einer synthetischen Computertomographie sind dazu nicht einmal die gewohnten Abläufe zu ändern.

Weitere vorteilhafte Ausführungsformen sind zu den nachfolgenden Unteransprüchen angegeben.

In einer denkbaren Ausführungsform des erfindungsgemäßen Verfahrens umfasst in dem Schritt des Ermittelns einer synthetischen Computertomographie die Abhängigkeit von der Computertomographie eine Abhängigkeit von einer mit der Computertomographie erfassten Strahlabschwächung durch die Knochen. Beispielsweise kann ein Abschwächungskoeffizient für Volumenelemente auf der Trajektorie des virtuellen Röntgenstrahls aus Messwerten der Computertomographie für die Volumenelemente entnommen und beim Registrieren den entsprechenden Volumenelementen des Knochens in der Magnetresonanztomographie zugeordnet werden. Denkbar sind auch davon ableitbare Werte wie beispielsweise die Knochendichte.

Auf vorteilhafte Weise geben die aus der Computertomographie gewonnenen Messwerte in Zusammenhang mit der Abschwächung der Röntgenstrahlung auch die Abschwächung der ionisierenden Strahlung bei der Therapie gut wieder. Indem diese über die Dauer der Therapie im Wesentlichen unveränderten Werte auf die Knochen in der jeweils aktuelle Magnetresonanztomographie registriert werden, kann die aktuelle Position aus der Magnetresonanztomographie in der synthetischen Computertomographie berücksichtigt werden, ohne jeweils den Patienten einer neuen Dosis bei einer Computertomographie auszusetzen oder durch einen patientenunabhängigen Schätzwert für den Knochen eine größere Sicherheitsmarge und Belastung vorsehen zu müssen.

In einer denkbaren Ausführungsform des erfindungsgemäßen Verfahrens weist in dem Schritt des Ermittelns einer synthetischen Computertomographie die Abhängigkeit von der Computertomographie eine Abhängigkeit von einer geometrischen Eigenschaft des Knochens auf. Als geometrische Eigenschaften sind dabei insbesondere Form und/oder Abmessungen anzusehen. Denkbar ist beispielsweise, dass der Knochen in der Magnetresonanztomographie bei der Berechnung der synthetischen Computertomographie skaliert oder verzerrt wird.

Auf vorteilhafte Weise wird die höhere Geometrietreue der Computertomographie und die Konstanz der Knochengeometrie genutzt, um die Geometrietreue der Magnetresonanztomographie als Grundlage der synthetischen Computertomographie zu verbessern.

In einer möglichen Ausführungsform des erfindungsgemäßen Verfahrens umfasst in dem Schritt Registrieren der in der Computertomographie segmentierten Knochen in der Magnetresonanztomographie weiterhin den Schritt, eine Geometrie der Magnetresonanztomographie in Abhängigkeit von dem registrierten Knochen zu korrigieren oder auch nur zu überprüfen. Mit anderen Worten, es wird beim Registrieren der Knochen deren im Wesentlichen unveränderliche Geometrie genutzt, um die Magnetresonanztomographie zu entzerren.

Die Computertomographie liefert eine Abbildung der Knochen, die im Wesentlichen geometrietreu ist, während eine Magnetresonanztomographie aufgrund statischer und dynamischer Magnetfeld-Inhomogenitäten nicht nur skaliert, sondern auch gekrümmt ist. Durch Vergleich der Knochen in Computertomographie und Magnetresonanztomographie in der Registrierung kann nicht nur der Knochen selbst korrigiert werden, sondern durch die Verzerrungen auf Magnetfeldfehler geschlossen werden und insbesondere die Geometrie der Magnetresonanztomographie in der Umgebung und/oder zwischen Knochen korrigiert werden. Denkbar ist es auch, dass lediglich die Geometrie überprüft wird und bei Abweichungen eine Warnmeldung an einen Nutzer ausgegeben wird.

Auf vorteilhafte Weise kann so die Magnetresonanztomographie zumindest teilweise entzerrt und so die Genauigkeit der darauf beruhenden synthetischen Computertomographie und Therapieplanung verbessert werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden.

Es zeigen:
- Fig. 1: eine schematische Darstellung eines Magnetresonanztomographen zur Ausführung des erfindungsgemäßen Verfahrens;
- Fig. 2: eine beispielhafte Systemumgebung zur Ausführung des Verfahrens;
- Fig. 3: einen schematischen Ablaufplan einer Ausführungsform des erfindungsgemäßen Verfahrens.

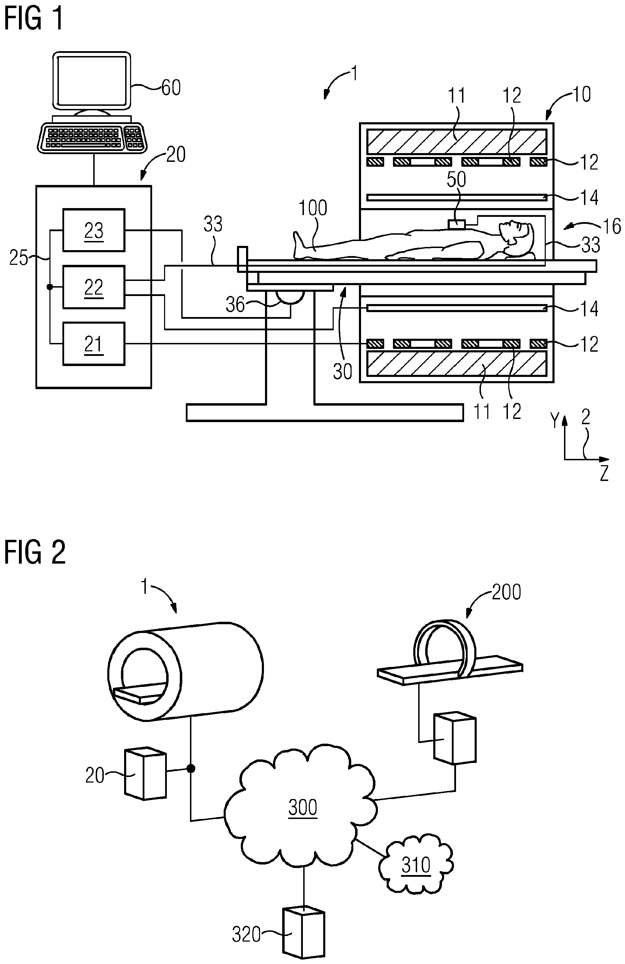

Fig. 1 zeigt eine schematische Darstellung einer Ausführungsform eines Magnetresonanztomographen 1 zum Ausführen des erfindungsgemäßen Verfahrens.

Die Magneteinheit 10 weist einen Feldmagneten 11 auf, der ein statisches Magnetfeld B0 zur Ausrichtung von Kernspins von Proben bzw. des Patienten 100 in einem Aufnahmebereich erzeugt. Der Aufnahmebereich zeichnet sich durch ein äußerst homogenes statisches Magnetfeld B0 aus, wobei die Homogenität insbesondere die Magnetfeldstärke bzw. den Betrag betrifft. Der Aufnahmebereich ist nahezu kugelförmig und in einem Patiententunnel 16 angeordnet, der sich in einer Längsrichtung 2 durch die Magneteinheit 10 erstreckt. Eine Patientenliege 30 ist in dem Patiententunnel 16 von der Verfahreinheit 36 bewegbar. Üblicherweise handelt es sich bei dem Feldmagneten 11 um einen supraleitenden Magneten, der magnetische Felder mit einer magnetischen Flussdichte von bis zu 3T, bei neuesten Geräten sogar darüber, bereitstellen kann. Für geringere Magnetfeldstärken können jedoch auch Permanentmagnete oder Elektromagnete mit normalleitenden Spulen Verwendung finden.

Weiterhin weist die Magneteinheit 10 Gradientenspulen 12 auf, die dazu ausgelegt sind, zur räumlichen Differenzierung der erfassten Abbildungsbereiche in dem Untersuchungsvolumen dem Magnetfeld B0 zeitlich und räumlich variable Magnetfelder in drei Raumrichtungen zu überlagern. Die Gradientenspulen 12 sind üblicherweise Spulen aus normalleitenden Drähten, die zueinander orthogonale Felder in dem Untersuchungsvolumen erzeugen können.

Die Magneteinheit 10 weist ebenfalls eine Körperspule 14 auf, die dazu ausgelegt ist, ein über eine Signalleitung zugeführtes Hochfrequenzsignal in das Untersuchungsvolumen abzustrahlen und von dem Patient 100 emittierte Resonanzsignale zu empfangen und über eine Signalleitung abzugeben.

Eine Steuereinheit 20 versorgt die Magneteinheit 10 mit den verschiedenen Signalen für die Gradientenspulen 12 und die Körperspule 14 und wertet die empfangenen Signale aus.

So weist die Steuereinheit 20 eine Gradientenansteuerung 21 auf, die dazu ausgelegt ist, die Gradientenspulen 12 über Zuleitungen mit variablen Strömen zu versorgen, welche zeitlich koordiniert die erwünschten Gradientenfelder in dem Untersuchungsvolumen bereitstellen.

Weiterhin weist die Steuereinheit 20 eine Hochfrequenzeinheit 22 auf, die ausgelegt ist, einen Hochfrequenz-Puls mit einem vorgegebenen zeitlichen Verlauf, Amplitude und spektraler Leistungsverteilung zur Anregung einer Magnetresonanz der Kernspins in dem Patienten 100 zu erzeugen. Dabei können Pulsleistungen im Bereich von Kilowatt erreicht werden. Die Anregungssignale können über die Körperspule 14 oder auch über eine lokale Sendeantenne in den Patienten 100 abgestrahlt werden.

Eine Steuerung 23 kommuniziert über einen Signalbus 25 mit der Gradientensteuerung 21 und der Hochfrequenzeinheit 22.

Fig. 2 zeigt eine beispielhafte Systemumgebung zur Ausführung des erfindungsgemäßen Verfahrens.

Der Magnetresonanztomograph 1 bzw. dessen Steuerung 20 steht in Verbindung mit einem Datennetzwerk 300. Das Datennetzwerk 300 kann beispielsweise ein LAN oder WAN wie ein IP-Netzwerk sein. Denkbar sind auch WAN-Netzwerke wie Mobilfunknetze. Das Datennetzwerk 300 ist vorzugsweise ausgelegt, Datenmengen wie sie bei Computertomographien oder Magnetresonanztomographien anfallen in Echtzeit oder nahezu Echtzeit zu übertragen.

Weiterhin ist ein Computertomograph 200, vorzugsweise ein Cone-Beam-CT, mit dem Datennetzwerk 300 verbunden. Der Computertomograph 200 ist ausgelegt, eine Computertomographie eines Patienten 100 zu erfassen, die Informationen zur Absorption von Röntgenstrahlen in Knochen des Patienten 100 umfasst und diese über das Datennetzwerk 300 zu übertragen. Darüber hinaus sind mit dem Datennetzwerk 300 Speicher- und Rechenressourcen wie Server 320 und/oder eine Anwendungs-Cloud 310 verbunden, sodass zwischen den beschriebenen Komponenten Bilddaten und Anweisungen zur Ausführung des erfindungsgemäßen Verfahrens ausgetauscht werden können.

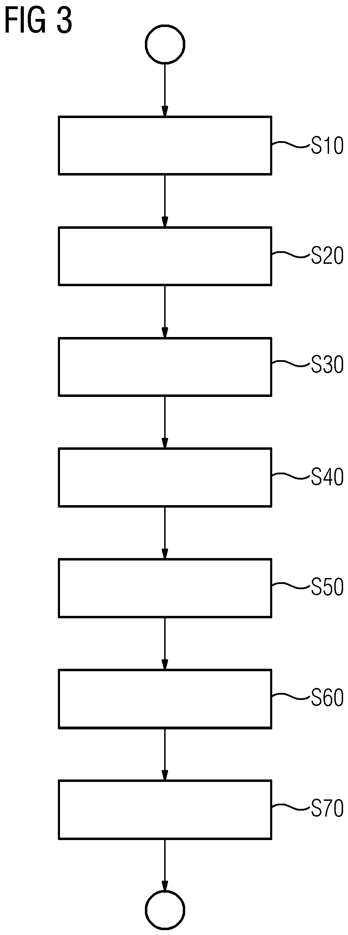

Fig. 3 zeigt einen schematischen Ablaufplan einer Ausführungsform des erfindungsgemäßen Verfahrens.

In einem Schritt S10 wird eine Computertomographie eines zu therapierenden Bereichs des Patienten 100 bereitgestellt. Beispielsweise kann die Computertomographie mittels des Computertomographen 200 erfasst und über das Datennetzwerk 300 zu einem Server 320 oder in eine Anwendungs-Cloud 310 zur Verarbeitung übertragen werden. Denkbar ist es aber auch, dass die Computertomographie im Lauf einer Behandlung bereits erfasst wurde und auf einem Server 320 gespeichert wurde. Im Schritt S10 des Verfahrens wird dann die Computertomographie zu der Steuerung über das Netzwerk 300 übertragen, auf der der nachfolgende Schritt S30 der Segmentierung ausgeführt wird. Denkbar ist es auch, dass die Computertomographie über ein Speichermedium wie ein Datenträger der ausführenden Steuerung bereitgestellt wird. Da die Knochen und insbesondere die Absorption sich nicht kurzfristig ändern, ist es auf vorteilhafte Weise nicht erforderlich, dass die Computertomographie unmittelbar vor einer Therapieplanung oder Bestrahlung jeweils neu erfasst wird.

In einer bevorzugten Ausführungsform wird dabei die Computertomographie von einem Cone-Beam-Computertomographen erfasst, der bei geringerer Strahlendosis eine besonders gute Darstellung der Knochen bietet.

In einem Schritt S20 wird eine Magnetresonanztomographie des zu therapierenden Bereichs des Patienten 100 bereitgestellt. Vorzugsweise wird die Magnetresonanztomographie mittels des Magnetresonanztomographen 1 erfasst und über das Datennetzwerk 300 zu einem Server 320 oder in eine Cloud 310 zur Verarbeitung übertragen. Da die Magnetresonanztomographie vorzugsweise genutzt wird, um die aktuelle Lage der Weichgewebe, insbesondere eines zu behandelnden Tumors, für die Behandlung zu erfassen, erfolgt der Schritt S20 vorzugsweise unmittelbar vor oder sogar während Behandlung oder zumindest bei gleicher Lage des Patienten wie nachfolgend bei der Behandlung.

In einem Schritt S30 werden die Knochen in der Computertomographie segmentiert. Darunter ist insbesondere das Erfassen der Konturen der Knochen in Abgrenzung zum umgebenden Gewebe in 2- oder 3-dimensionalen Koordinaten zu verstehen. Das Segmentieren in der Computertomographie erfolgt vorzugsweise anhand des unterschiedlichen Absorptionskoeffizienten von Knochen und umgebendem Gewebe.

Weiterhin werden in einem weiteren Schritt S40 die Knochen in der Magnetresonanztomographie in gleicher Weise segmentiert. Das Segmentieren erfolgt jedoch aufgrund der Kontrastunterschiede in der Magnetresonanztomographie, beispielsweise unterschiedlicher Kernspindichte oder Relaxationszeiten. Das Segmentieren erfolgt dabei vorzugsweise so, dass die Position der Knochen im nachfolgenden Schritt einander zugeordnet werden kann.

Das Segmentieren kann dabei beispielsweise auf der gleichen Steuerung erfolgen, beispielsweise einem Anwendungsserver in der Anwendungs-Cloud 310. Das Segmentieren kann auch durch eine künstliche Intelligenz auf dem Server 320 oder in der Anwendungs-Cloud 310 erfolgen, die beispielsweise mit Trainingsdaten in einem Deep-Learning-Algorithmus trainiert wurden. Denkbar ist es aber auch, dass das Segmentieren bereits beim Erfassen der Abbildungen in dem Computertomographen 200 bzw. dem Magnetresonanztomographen 1 erfolgt.

In einem weiteren Schritt S50 werden die in der Computertomographie segmentierten Knochen in der Magnetresonanztomographie registriert. Darunter ist zu verstehen, dass die in der Computertomographie segmentierten Knochen den in der Magnetresonanztomographie segmentierten Knochen zugeordnet werden. Dabei werden aus der Computertomographie ermittelten Eigenschaften der Knochen auf die Knochen in der Magnetresonanztomographie übertragen bzw. diesen zugeordnet. Dies betrifft insbesondere Eigenschaften, die wesentlich für die Therapieplanung sind, wie Absorption der Röntgenstrahlen durch die Knochen oder deren Geometrie wie Abmessungen und Form. Denkbar ist es beispielsweise, dass die Knochen in der Magnetresonanztomographie entsprechend der Computertomographie in ihrer Geometrie beim Registrieren korrigiert bzw. entzerrt werden. Dabei kann auch die Umgebung der Knochen in der Magnetresonanztomographie entsprechend korrigiert werden.

Das Registrieren der Computertomographie erfolgt dabei beispielsweise auf einem Server 320 oder in einer Anwendungs-Cloud 310. Auch hier ist die Anwendung einer künstlichen Intelligenz denkbar, die mit Beispieldaten trainiert wurde.

In einem weiteren Schritt S60 wird eine synthetische Computertomographie aus der Magnetresonanztomographie in Abhängigkeit der registrierten Knochen der Computertomographie ermittelt. Vorzugsweise erfolgt das Ermitteln der synthetischen Computertomographie auf der gleichen Steuerung bzw. dem gleichen Server 320 oder in der Anwendungs-Cloud 310, in der das Registrieren erfolgte. Es ist aber auch denkbar, dass die Magnetresonanztomographie, in der die Knochen der Computertomographie mit einer oder mehreren Eigenschaften registriert bzw. zugeordnet wurden, zu einer anderen Steuerung über das Datennetzwerk 300 übermittelt werden.

Die Magnetresonanztomographie liefert dabei aktuelle Daten zur Geometrie bzw. Anordnung der unterschiedlichen Gewebe, die auch eine bessere Unterscheidung der Gewebetypen z.B. in gesundes und krankhaftes Gewebe erlaubt. Für die Knochendichte bzw. Absorption der Röntgenstrahlung bietet hingegen die Computertomographie bessere Daten. Auch kann die Computertomographie aufgrund der besseren Geometrietreue Daten zur Korrektur von geometrischen Verzerrungen der Knochen und Umgebung in der Magnetresonanztomographie aufgrund Inhomogenitäten der Magnetfelder liefern.

Mit den geometrischen, gegebenen Falls durch die Computertomographie korrigierten Informationen, Messwerten für die Absorption der Knochen aus der Computertomographie und Annahmen oder Messwerten der Computertomographie zur Absorption der Weichgewebe kann die Absorption für einen virtuellen Röntgenstrahl unter unterschiedlichen Winkeln simuliert und daraus eine synthetische Computertomographie ermittelt werden.

In einem weiteren Schritt S70 wird ein Therapieplan in Abhängigkeit von der synthetischen Computertomographie erstellt. Dabei können die gleichen Verfahren, Werkzeuge und Kontrollen genutzt werden, wie sie bereits aufgrund konventioneller Computertomographie etabliert sind. Der Patient 100 profitiert dabei von der besseren Abbildung der Weichgewebe und der geringeren Strahlendosis von der Magnetresonanztomographie. Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Verfahren zur Therapieplanung für eine Strahlentherapie mittels eines Magnetresonanztomographen (1) und eines Computertomographen (200), wobei das Verfahren die Schritte aufweist
(S10) Bereitstellen einer Computertomographie eines zu therapierenden Bereichs eines Patienten (100);
(S20) Erfassen einer Magnetresonanztomographie des zu therapierenden Bereichs mit dem Magnetresonanztomographen (1); (S30) Segmentieren von Knochen in der Computertomographie; (S40) Segmentieren der Knochen in der Magnetresonanztomographie;
(S50) Registrieren der in der Computertomographie segmentierten Knochen in der Magnetresonanztomographie;
(S60) Ermitteln einer synthetischen Computertomographie aus der Magnetresonanztomographie in Abhängigkeit der registrierten Knochen der Computertomographie;
(S70) Ermitteln eines Therapieplans in Abhängigkeit von der synthetischen Computertomographie.

2. Verfahren nach Anspruch 1, wobei das Bereitstellen der Computertomographie einen Schritt des Erfassens einer Computertomographie des zu therapierenden Bereichs mit einem Cone-Beam-Computertomographen (200) aufweist.

3. Verfahren nach Anspruch 1 oder 2, wobei in dem Schritt des Ermittelns einer synthetischen Computertomographie die Abhängigkeit von der Computertomographie eine Abhängigkeit von einer mit der Computertomographie erfassten Strahlabschwächung durch die Knochen umfasst.

4. Verfahren nach einem der vorhergehenden Ansprüche, wobei in dem Schritt des Ermittelns einer synthetischen Computertomographie die Abhängigkeit von der Computertomographie eine Abhängigkeit von einer geometrischen Eigenschaft des Knochens umfasst.

5. Verfahren nach Anspruch 4, wobei in dem Schritt Registrieren der in der Computertomographie segmentierten Knochen in der Magnetresonanztomographie den Schritt umfasst, eine Geometrie der Magnetresonanztomographie in Abhängigkeit von dem registrierten Knochen zu korrigieren.

6. Computerprogrammprodukt, welches direkt in einen Prozessor einer programmierbaren Steuerung ladbar ist, mit Programmcode-Mitteln, um alle Schritte eines Verfahrens nach einem der Ansprüche 1 bis 5 auszuführen, wenn das Programmprodukt auf der Steuerung ausgeführt wird.
